# EUROPEAN PATENT APPLICATION

(11) **EP 4 400 579 A1**
(43) Date of publication of application: **17.07.2024**
(21) Application number: 22867394.3
(22) Date of filing: 08.09.2022
(51) Int. Cl.: C12N 1/20, C12Q 1/02, A23C 9/123

(54) **LACTIC ACID BACTERIA, LACTIC ACID BACTERIA STARTER, FERMENTED MILK, FERMENTED MILK PRODUCTION METHOD, AND SCREENING METHOD OF LACTIC ACID BACTERIA**

(30) Priority: 09.09.2021 JP 2021147152
(71) Applicant: MEIJI CO., LTD, Chuo-ku Tokyo 104-8306 (JP)
(72) Inventor: YAMAMOTO Eri, Hachioji-shi, Tokyo 192-0919 (JP); HONME Yoshiko, Hachioji-shi, Tokyo 192-0919 (JP); TOYAMA Emi, Hachioji-shi, Tokyo 192-0919 (JP); GOTO Hirofumi, Hachioji-shi, Tokyo 192-0919 (JP)
(74) Representative: TBK
(86) International application number: PCT/JP2022/033649
(87) International publication number: WO 2023/038073

(57) **Abstract**

The present invention relates to a lactic acid bacterium in which an ATP synthase complex is functionally inhibited.

## Description

### [Technical Field]

The present invention relates to lactic acid bacterium, a lactic acid bacterium starter, fermented milk, and a method for producing fermented milk, and more particularly to lactic acid bacterium, a lactic acid bacterium starter and fermented milk comprising the same, and a method for producing fermented milk, and further a method for screening lactic acid bacterium.

### [Background Art]

For example, in Japan's "Ministerial Ordinance on Milk and Milk Products Concerning Compositional Standards, etc. (Ministerial Ordinance on Milk and Milk Products)", fermented milk is defined as "products which are obtained by fermenting milk, or milk, etc. comprising an equal or greater amount of milk solids-not-fat with lactic acid bacteria or yeast and then forming a paste or liquid, or the frozen product." Representative examples of such fermented milk include yogurt such as set type yogurt (solid fermented milk), soft type yogurt (pasty fermented milk), and drink type yogurt (liquid fermented milk). Note that, for example, in the "Codex Alimentarius (FAO (Food and Agriculture Organization of the United Nations)/WHO (World Health Organization))", which is a food standard shared by the international community, yogurt is defined as "any food that is made through the process of lactic acid fermentation combining Lactobacillus delbrueckii subsp. bulgaricus and Streptococcus thermophilus (S. thermophilus)." Traditionally, yogurt is generally characterized by adding these two types of bacteria as starters to raw material milk and fermenting the lactose in the raw material milk, primarily characterized by its sourness due to the lactic acid produced during fermentation.

However, lactic acid bacteria such as Streptococcus thermophilus are capable of producing lactic acid through fermentation even at low temperatures. Therefore, in the fermented products after fermentation (for example, fermented milk such as yogurt), during storage, even under low-temperature conditions, an excessive production of lactic acid leads to an increase in acidity (that is, decrease in pH), posing a problem of compromising its flavor.

Consequently, research efforts have been undertaken to inhibit the acid production in such lactic acid bacteria at low temperatures. For instance, Japanese Patent Application Publication No. Hei 7-236416 (PTL 1) describes the use of acid production inhibition strains of Lactobacillus delbrueckii subsp. bulgaricus and viscosity producing strains of Streptococcus salivarius subsp. thermophilus to obtain fermented milk with minimal increase in acidity during low-temperature storage. Furthermore, Japanese Patent No. 4331309 (PTL 2) has been granted for a lactic acid bacterial strain belonging to the low-temperature-sensitive Streptococcus thermophilus, which coagulates 12% reduced skim milk with 0.05% yeast extract addition within 3 hours at 37 to 43°C but does not coagulate 12% reduced skim milk with 0.05% yeast extract addition within 3 days at 15 to 25°C. Specifically, Streptococcus thermophilus SBT0144 is described as one of the lactic acid bacterial strains.

Moreover, Japanese Patent Application Publication No. 2001-95561 (PTL 3) describes mutant strains of Lactobacillus delbrueckii subsp. bulgaricus with reduced cell membrane-bound adenosine triphosphatase (ATPase) activity. In these mutant strains, a decrease in pH during the fermentation process leads to intracellular pH reduction due to the retention of H⁺ within the cells, inhibiting growth (that is, inducing so-called apoptosis) . As a result, the production of lactic acid decreases. Furthermore, Japanese Patent Application Publication No. Hei 9-9954 (PTL 4) describes Lactococcus lactis subsp. lactis mutant strains with reduced ATPase activity. It is stated that according to these mutant strains, a decrease in pH during the fermentation process leads to inhibition of their growth, resulting in a reduced production of lactic acid. Additionally, Japanese Patent Application Publication No. Hei 7-123977 (PTL 5) states that lactic acid bacteria of the genus Lactobacillus with low ATPase activity are used to inhibit the increase in lactic acid acidity during long-term low-temperature storage.

### [Citation List]

### [Patent Literature]

[PTL 1] Japanese Patent Application Publication No. Hei 7-236416
[PTL 2] Japanese Patent No. 4331309
[PTL 3] Japanese Patent Application Publication No. 2001-95561
[PTL 4] Japanese Patent Application Publication No. Hei 9-9954
[PTL 5] Japanese Patent Application Publication No. Hei 7-123977

### [Summary of Invention]

### [Technical Problem]

However, factors contributing to the acid production at low temperatures in fermented milk by lactic acid bacteria, other than the aforementioned ATPase activity, have not been reported. Additionally, when lactic acid bacteria with reduced ATPase activity are used, it has been observed by the present inventors that although the acid production at low temperatures in the resulting fermented milk is indeed reduced, and the decrease in pH is suppressed, there is a problem in that it takes time for fermentation to complete (for example, until the pH reaches 4.7 or 4.6 or lower), and there is a decrease in the viable bacterial count of lactic acid bacteria during low-temperature storage.

The present invention has been made in view of the above problems posed by the related art, and aims to provide lactic acid bacterium which allows for obtaining fermented milk in which the decrease in pH during low-temperature storage is suppressed, and the viable bacterial count of the lactic acid bacteria is sufficiently maintained even by the low-temperature storage, a lactic acid bacterium starter and fermented milk comprising the same, and a method for producing fermented milk, and further a method for screening lactic acid bacterium.

### [Solution to Problem]

The present inventors have diligently conducted research in order to achieve the above objects. Firstly, in order to explore factors contributing to low-temperature acid production in lactic acid bacteria, they conducted genome analysis between lactic acid bacteria capable of suppressing pH decrease during low-temperature storage (low acid-producing lactic acid bacteria) and lactic acid bacteria incapable of suppressing pH decrease (high acid-producing lactic acid bacteria). In this genome analysis, multiple mutations, including amino acid mutations (loss-of-function mutations), common to low acid-producing lactic acid bacteria were extracted. Subsequently, recombinant strains of low acid-producing lactic acid bacteria and high acid-producing lactic acid bacteria were prepared to verify whether each mutation contributed to acid production. As a result, it has been found that mutations in the gene encoding the ε subunit (ε subunit gene) that constitutes the ATP synthase complex (ATP synthase epsilon chain) contribute to low acid production in lactic acid bacteria. Furthermore, since the viable bacterial count was sufficiently maintained even in low-temperature storage, lactic acid bacteria with loss-of-function mutations in the ε subunit gene were found to exhibit low acid production through a mechanism distinct from the decrease in ATPase activity, as described in conventional PTLs 3 to 5.

Therefore, the present inventors have found, based on these findings, that in lactic acid bacterium where the ε subunit-comprising ATP synthase complex is functionally inhibited, it is possible to obtain fermented milk where pH decrease is suppressed even with low-temperature storage, and viable bacterial counts of lactic acid bacteria are sufficiently maintained even with that low-temperature storage, leading to the completion of the present invention.

The aspects of such present invention are as follows.
[1] A lactic acid bacterium in which an ATP synthase complex is functionally inhibited.
[2] The lactic acid bacterium according to [1], wherein an ε subunit of the ATP synthase complex is functionally inhibited.
[3] The lactic acid bacterium according to [1] or [2], which is a lactic acid bacterium of the genus Streptococcus.
[4] The lactic acid bacterium according to [3], which is a lactic acid bacterium belonging to Streptococcus thermophilus.
[5] The lactic acid bacterium according to [4], possessing a prtS gene.
[6] The lactic acid bacterium according to any one of [1] to [5], wherein the function inhibition of the ATP synthase complex is a loss-of-function mutation in an ε subunit gene.
[7] The lactic acid bacterium according to any one of [1] to [6], wherein the function inhibition of the ATP synthase complex is a decreased expression of an ε subunit protein.
[8] A lactic acid bacterium composition comprising the lactic acid bacterium according to any one of [1] to [7].
[9] The lactic acid bacterium composition according to [8], wherein the lactic acid bacterium is a lactic acid bacterium of the genus Streptococcus, the composition further comprising: lactic acid bacterium of the genus Lactobacillus.
[10] The lactic acid bacterium composition according to [8] or [9], which is a lactic acid bacterium starter.
[11] The lactic acid bacterium composition according to [8] or [9], which is fermented milk.
[12] A method for producing fermented milk comprising: a fermentation step of adding the lactic acid bacterium according to any one of [1] to [7] or the lactic acid bacterium composition according to any one of [8] to [11] to a formula milk comprising raw material milk, followed by fermentation to thereby obtain fermented milk.
[13] The method for producing fermented milk according to [12], wherein the lactic acid bacterium is a lactic acid bacterium of the genus Streptococcus, the method further comprising: adding lactic acid bacterium of the genus Lactobacillus to the formula milk.
[14] A method for screening lactic acid bacterium, comprising: a selection step of selecting lactic acid bacterium with low acid production using function inhibition of an ATP synthase complex as an indicator.
[15] A method for producing fermented milk comprising: a fermentation step of adding lactic acid bacterium selected by the method for screening lactic acid bacterium according to [14] to a formula milk comprising raw material milk, followed by fermentation to thereby obtain fermented milk.

### [Advantageous Effects of Invention]

The present invention makes it possible to provide lactic acid bacterium which allows for obtaining fermented milk in which the decrease in pH during low-temperature (such as 10°C) storage is suppressed, and the viable bacterial count of the lactic acid bacteria is sufficiently maintained even by the low-temperature storage, a lactic acid bacterium starter and fermented milk comprising the same, and a method for producing fermented milk, and further a method for screening lactic acid bacterium.

### [Brief Description of Drawings]

Fig. 1 is a graph illustrating the fermentation time (minutes) for recombinant strains, P2101201 strain, and OLS4803 strain in Test Example 3.
Fig. 2 is a graph illustrating the time course of pH for recombinant strains, P2101201 strain, and OLS4803 strain during storage at 10°C in Test Example 3.
Fig. 3 is a graph illustrating the time course of viable bacterial count for recombinant strains, P2101201 strain, and OLS4803 strain during storage at 10°C in Test Example 3.

### [Description of Embodiments]

Hereinafter, the present invention will be described in detail with reference to preferred embodiments thereof.

### <Lactic Acid Bacteria>

In the present invention, the term "lactic acid bacterium (bacteria)" refers to a collective term for microorganisms capable of assimilating glucose and producing lactic acid at a yield of 50% or more based on sugar, and as physiological characteristics, they are gram-positive cocci or rods, non-motile, often lacking the ability to form spores (although some lactic acid bacteria, such as Bacillus coagulans, have the ability to form spores), and possess features such as being catalasenegative.

Examples of lactic acid bacteria of the present invention include lactic acid bacteria of the genus Streptococcus, lactic acid bacteria of the genus Lactobacillus (such as Lactobacillus delbrueckii (especially Lactobacillus delbrueckii subsp. bulgaricus (Bulgarian bacterium), Lactobacillus delbrueckii subsp. delbrueckii, Lactobacillus delbrueckii subsp. lactis, Lactobacillus delbrueckii subsp. indicus, Lactobacillus delbrueckii subsp. sunkii, Lactobacillus delbrueckii subsp. jakobsenii, and the like), Lactobacillus helveticus, Lactobacillus acidophilus, Lactobacillus johnsonii, Lactobacillus gasseri, Lactobacillus paragasseri, Lactobacillus amylovorus, and Lactobacillus crispatus), lactic acid bacteria of the genus Lacticaseibacillus, lactic acid bacteria of the genus Lactiplantibacillus, lactic acid bacteria of the genus Liquorilactobacillus, lactic acid bacteria of the genus Latilactobacillus, lactic acid bacteria of the genus Ligilactobacillus, lactic acid bacteria of the genus Limosilactobacillus, lactic acid bacteria of the genus Lentilactobacillus, lactic acid bacteria of the genus Levilactobacillus, lactic acid bacteria of the genus Pediococcus, lactic acid bacteria of the genus Leuconostoc, and lactic acid bacteria of the genus Lactococcus. Among these, lactic acid bacteria of the genus Streptococcus are preferable as the lactic acid bacteria of the present invention.

### (Lactic Acid Bacteria of the Genus Streptococcus)

Lactic acid bacteria of the genus Streptococcus are aerobic, Gram-positive cocci. Examples of lactic acid bacteria belonging to the genus Streptococcus include Streptococcus thermophilus and Streptococcus salivarius. Among these lactic acid bacteria of the present invention, it is preferable to belong to Streptococcus thermophilus.

Streptococcus thermophilus, also referred to as thermophilus bacteria, is a streptococcus capable of producing lactic acid from lactose, thus being a lactic acid bacteria. In the present specification, lactic acid bacteria belonging to Streptococcus thermophilus are sometimes referred to as "S. thermophilus."

The lactic acid bacterium of the present invention, in the case of S. thermophilus, preferably possess the prtS gene. By employing S. thermophilus that possesses the prtS gene, it is possible to shorten the fermentation time until completion and further trend towards obtaining fermented milk with a more preferable flavor. In the present invention, the term "prtS gene" refers to a gene encoding a cell-wall-bound serine protease that degrades casein. Additionally, in the present invention, the possession of the prtS gene by S. thermophilus can be determined, for example, by selecting a highly conserved sequence among the prtS genes of S. thermophilus obtained from public databases (such as GenBank), amplifying a portion of the prtS gene by PCR using a primer set prepared based on the selected sequence, and confirming the desired PCR product. As an example of such a primer set, the primer set composed of Fprimer and Rprimer described in the following examples can be mentioned, but this is not limiting.

### (ATP Synthase Complex)

The lactic acid bacterium of the present invention is lactic acid bacterium in which the ATP synthase complex is functionally inhibited (including lactic acid bacteria in which the ATP synthase complex is artificially functionally inhibited). The "ATP synthase complex" is a protein complex composed of multiple constituent proteins, with a molecular weight exceeding 500,000, and possesses enzyme activity to synthesize ATP from ADP and inorganic phosphate. As known constituent proteins of the ATP synthase complex, eight proteins are known, namely the A subunit, B subunit, C subunit, α subunit, β subunit, γ subunit, δ subunit, and ε subunit.

The amino acid sequence of each constituent protein of the ATP synthase complex and the corresponding base sequence encoding it can be obtained from public databases (for example, GenBank). For example, the amino acid sequence of each constituent protein derived from S. thermophilus and the base sequence (base sequence of the gene) encoding it are typically represented by the sequences listed in Table 1 below. Table 1 shows the gene name of each constituent protein derived from S. thermophilus and the sequence number indicating its typical base sequence, as well as sequence numbers indicating the typical amino acid sequences for each constituent protein derived from S. thermophilus. Note that even for constituent proteins that have not undergone mutations, individual variations in sequences can occur due to polymorphism and other factors. The ATP synthase complex functions in a wide range of lactic acid bacteria, not limited to S. thermophilus. For example, among lactic acid bacteria of the genus Streptococcus, it exhibits almost 100% (at least 90% or more, more preferably 93% or more) high identity.

**[Table 1]**

| Subunit Name | Gene Name | Base Sequence SEQ ID NO | Amino Acid Sequence SEQ ID NO |
|---|---|---|---|
| A Subunit | atpB | 1 | 2 |
| B Subunit | atpF | 3 | 4 |
| C Subunit | atpE | 5 | 6 |
| α Subunit | atpA | 7 | 8 |
| β Subunit | atpD | 9 | 10 |
| γ Subunit | atpG | 11 | 12 |
| δ Subunit | atpH | 13 | 14 |
| ε Subunit | atpC | 15 | 16 |

### (Function Inhibition of ATP Synthase Complex)

In the present invention, "function inhibition of ATP synthase complex" includes a decrease in activity, including the inactivation of the ATP synthase complex, and the decreased expression of the ATP synthase complex. As for the function inhibition of the ATP synthase complex, it may be a function inhibition of the entire complex or a function inhibition of one or more combinations of the constituent proteins, but it is preferable to include at least the function inhibition of the A subunit and/or ε subunit, and it is more preferable to include at least the function inhibition of the ε subunit.

### [Reduced Activity of ATP Synthase Complex]

The term "reduced activity of the ATP synthase complex" refers to a reduction in the inherent activity of the ATP synthase complex, typically meaning that its activity level is lower than that of the control when compared. Additionally, the "reduced activity of the ATP synthase complex" includes both the inactivation and the reduction of activity of a portion or all of the ATP synthase complex.

### <Direct Activity Reduction of ATP Synthase Complex>

In the present invention, there are no particular limitations on the method for directly confirming the "reduced activity of the ATP synthase complex" of lactic acid bacteria. For example, a method can be employed where a protein sample is prepared from the target lactic acid bacteria, and NADH is added thereto in the presence of a fluorescent pH indicator (such as luciferase) or the like, and changes in fluorescence due to proton (H⁺) transport activity in ATP synthase activity are measured and compared to the activity in the control. As an example of such a measurement method, the method described in Suzuki et al., 2007 PNAS, Vol. 104, no. 52, 20776-20781 can be mentioned. More specifically, a method can be employed where the above protein sample is added in an appropriate amount to a PA3 buffer comprising 2.5 mM potassium phosphate, 0.5 mM ADP, and 2.5 mM NaCl (10 mM Hepes/KOH (pH 7.5), 10% Glycerol, 5 mM MgCl₂) to prepare a reaction solution, and an appropriate amount of luciferase is added to the reaction solution, and the reaction is initiated by adding NADH to a final concentration of 1.7 mM, and the fluorescence is measured to determine the change in fluorescence as a measurement of proton transport activity, that is, ATP synthase activity. The amount of protein sample, reaction solution, and luciferase can be adjusted as appropriate depending on the conditions of the sample and the like. Moreover, there are no particular limitations on the method for preparing a protein sample from lactic acid bacteria, and a known method or a method similar thereto can be appropriately employed.

If the above-described activity is reduced in comparison with the control, it can be determined that the activity of the ATP synthase complex is reduced, that is, the ATP synthase complex is functionally inhibited in the lactic acid bacteria. The criteria for this determination are not particularly limited, but, for example, if the activity is 4 SD or less, 3 SD or less, or 2 SD or less of the average activity in the control (SD: standard deviation), it may be determined that ATP synthase activity is low, meaning that the activity of the ATP synthase complex is reduced in the lactic acid bacteria.

As a control in this case, examples include lactic acid bacteria known to have no reduced activity of the ATP synthase complex, more specifically, for example, Streptococcus thermophilus OLS4496 (hereinafter referred to as "OLS4496 strain" as needed) identified by accession number NITE BP-02875. The OLS4496 strain has been deposited with (1) identification label: Streptococcus thermophilus OLS4496, (2) accession number: NITE BP-02875, (3) accession date: February 5, 2019 at (4) depositary institution: National Institute of Technology and Evaluation NITE Patent Microorganisms Depositary (postal code 292-0818, Room 122, 2-5-8 Kazusa Kamatari, Kisarazu City, Chiba Prefecture).

### <Genetic Mutation of ATP Synthase Complex Constituent Protein>

The reduced activity of the ATP synthase complex is typically due to a loss-of-function mutation in the genes of the constituent proteins of the complex. Therefore, in the present invention, as to the "reduced activity of the ATP synthase complex" of lactic acid bacteria, the detection of a "loss-of-function mutation in the genes of the constituent proteins" makes it possible to indirectly determine that the activity of the ATP synthase complex is decreased, that is, the ATP synthase complex is functionally inhibited in the detected mutated lactic acid bacteria.

In the present invention, the term loss-of-function mutation refers to a mutation involving amino acid mutations, and these amino acid mutations include substitution, deletion, insertion, and/or addition of one or more amino acids. Such loss-of-function mutations are not particularly limited, but include, for example, missense mutations, nonsense mutations, or frame-shift mutations caused by substitution of one or more bases; and complete or partial gene deletions or insertions, and preferably mutations involving the destruction of genes (such as mutations involving the substitution and/or deletion of 1 or more, 5 or more, or 10 or more (preferably 20, 30, 40, 50, or 60 or more amino acids)).

Among these, the lactic acid bacterium of the present invention is preferably such that the loss-of-function mutation in the genes of the constituent proteins (that is, the function inhibition of the ATP synthase complex) is at least one of a loss-of-function mutation in the A subunit gene and a loss-of-function mutation in the ε subunit gene, and more preferably such that it is a loss-of-function mutation in the ε subunit gene.

The A subunit, which is one of the constituent proteins, is the catalytically active part of the ATP synthase complex, and when a loss-of-function mutation (amino acid mutation) occurs in the gene encoding this A subunit, it leads to reduced activity of the ATP synthase complex. More specifically, the loss-of-function mutation in the A subunit gene is preferably a loss-of-function mutation in the base sequence set forth in SEQ ID NO: 1.

Examples of loss-of-function mutations in the A subunit gene include, but are not limited to, substitutions of bases that cause the substitution of the first valine of the amino acid sequence set forth in SEQ ID NO: 2 or the valine corresponding to the first valine of the amino acid sequence set forth in SEQ ID NO: 2 with a different amino acid (such as alanine).

The ε subunit, which is one of the constituent proteins, contributes to the binding with ATP within the ATP synthase complex. Therefore, when a loss-of-function mutation (amino acid mutation) occurs in the gene encoding this ε subunit, it causes a reduced activity of the ATP synthase complex (Krah A. et al., 2018, rsob.royalsocietypublishing.org, Open Biol. 8: 170275, etc.). More specifically, the loss-of-function mutation in the ε subunit gene is preferably a loss-of-function mutation in the base sequence set forth in SEQ ID NO: 15.

Examples of loss-of-function mutations in the ε subunit gene include, but are not limited to, base substitutions, deletions, or insertions (more preferably deletions or insertions) that cause the substitution (missense) or deletion of 10 or more (preferably, 20, 30, 40, 50, or 60 or more, further preferably 20, 30, 40, 50, or 60 or more of the amino acids following the 86th glutamic acid in the amino acid sequence set forth in SEQ ID NO: 16) amino acids among the amino acid sequence set forth in SEQ ID NO: 16 or the amino acid sequence corresponding to the amino acid sequence set forth in SEQ ID NO: 16; base substitutions that cause the substitution of the 30th glycine in the amino acid sequence set forth in SEQ ID NO: 16 or the glycine corresponding to the 30th glycine in the amino acid sequence set forth in SEQ ID NO: 16 with a different amino acid (such as alanine); and base substitutions that cause at least one of the substitutions of at least any of the amino acids, namely the 86th glutamic acid, 95th arginine, and 129 arginine in the amino acid sequence set forth in SEQ ID NO: 16, or an amino acid corresponding to any of these amino acids, with a different amino acid (such as alanine).

Note that in the present invention, the amino acid sequence or amino acid "corresponding" to an amino acid sequence or amino acid refers to an amino acid sequence or amino acid that, when aligning amino acid sequences using amino acid sequence analysis software (such as GENETYX-MAC or Sequencher), BLAST (Basic Local Alignment Search Tool at the National Center for Biological Information), for example, with parameters set to default values (that is, initial settings), or the like, aligns with the reference amino acid sequence (such as the amino acid sequence set forth in SEQ ID NO: 2 or SEQ ID NO: 16) or amino acid (such as the first valine of the amino acid sequence set forth in SEQ ID NO: 2 or the 30th glycine in the amino acid sequence set forth in SEQ ID NO: 16).

In the present invention, the method for detecting a "loss-of-function mutation in the genes of the constituent proteins" is not particularly limited, but includes, for example, the following methods. Note that "mutation detection" in a gene in the present invention principally signifies detecting mutations on the genomic DNA. However, since the mutations on this genomic DNA are reflected in the changes of bases in transcription products and changes of amino acids in translation products, it also encompasses detecting these changes in these transcription and translation products (that is, indirect detection).

An embodiment of the above detection method includes a method involving directly determining the base sequences of the gene regions of the constituent proteins of the ATP synthase complex and comparing it with a control to detect mutations. In the present invention, the "gene regions of the constituent proteins" refers to a specific region on the genomic DNA comprising the gene of the constituent proteins. These region each independently include, apart from the translation region, non-translation regions such as the expression control regions of each gene (such as promoter regions and enhancer regions) and the 5'-terminus untranslated regions of each gene.

In the present embodiment, first, DNA samples are prepared from the target lactic acid bacteria. Examples of DNA samples include genomic DNA samples, and cDNA samples prepared by reverse transcription from RNA. There are no particular limitations on the method for extracting genomic DNA or RNA from the lactic acid bacteria and the method for preparing cDNA, and for each of them, any known method or a method similar thereto can be appropriately employed. Then, DNA including the gene regions of the constituent proteins is isolated, and the base sequence of the isolated DNA is determined. The isolation of this DNA can be performed, for example, using a pair of oligonucleotide primers designed to encompass all or part of the gene regions of the constituent proteins by PCR or the like with genomic DNA or RNA as a template. The determination of the base sequence of the isolated DNA can be performed by a method known to those skilled in the art, such as the Maxam-Gilbert method or the Sanger method. Furthermore, it is also possible to determine the base sequence using next-generation sequencers capable of rapidly and comprehensively reading out the base sequence of the genes by preparing a library from the DNA sample.

By comparing the determined DNA or cDNA base sequence with the control, it is possible to judge the presence or absence of mutations in the gene regions of the constituent proteins in the ATP synthase complex of the target lactic acid bacteria. The control at this time can include the known base sequences of the genes of the constituent proteins, which can be obtained from public databases such as GenBank. More specifically, for example, each base sequence presented in Table 1 can be mentioned.

Examples of other embodiments of the aforementioned detection method include the following methods:
a method utilizing restriction fragment length polymorphism (RFLP) where DNA comprising all or part of the gene regions of the constituent proteins is amplified, and the size of the DNA fragments of the amplification products by restriction enzymes is compared with a control, or PCR-RFLP method;
the PCR-SSCP (single-strand conformation polymorphism) method where the amplification products are dissociated into single-stranded DNA, separated on a nondenaturing gel, and compared in mobility on the gel with the control;
the denaturant gradient gel electrophoresis (DGGE) method where the amplification products are separated on a gel with a concentration gradient of DNA denaturant, and compared in mobility on the gel with the control;
the MALDI-TOF/MS method where an "oligonucleotide primer having bases on the 1-base 3'-side among all or part of the bases of the gene regions of the constituent proteins and a base sequence complementary to the base sequence on the 3'-side thereof" is used with the above DNA sample as a template to perform ddNTP primer extension reaction, and the genotype determined by mass measurement is compared with a control;
the invader method where the above DNA sample is hybridized with an oligonucleotide probe composed of 5' - "all or part of the bases of the gene regions of the constituent proteins and a base sequence complementary to the base sequence of the 5'-side thereof" - "a base sequence not hybridizing with all or part of the bases on the 1-base 3'-side of the gene regions of the constituent proteins and the base sequence of the 3'-side thereof" - 3' (flap) and an "oligonucleotide probe having all or part of the bases of the gene regions of the constituent proteins and a base sequence complementary to the base sequence on the 3'-side thereof," and then the amount of released flap cut with single-stranded DNA cleavage enzyme is measured based on the oligonucleotide probe labeled with reporter fluorescent dye and quencher fluorescent dye, and compared with the control;
the pyrosequencing method where the amplification products are dissociated into single strands and only one of the strands is separated, and extension reaction is performed base by base from the vicinity of all or part of the bases of the gene regions of the constituent proteins, and the pyrophosphate produced at that time is measured and compared with the control; and
the AcycloPrime method where in the presence of fluorescent-labeled nucleotides, a one-base extension reaction is performed with an "oligonucleotide primer having bases on the 1-base 3'-side among all or part of the bases of the gene regions of the constituent proteins and a base sequence complementary to the base sequence on the 3'-side thereof" using the above amplification products as a template, and the fluorescence anisotropy is measured and compared with the control, and the SNuPE method where the type of base used in the one-base extension reaction is determined and compared with the control.

As a control at this time, a DNA sample prepared in the same way from lactic acid bacteria known to have no genetic mutations in each constituent protein can be mentioned, and more specifically, for example, a DNA sample prepared in the same way from OLS4496 strain can be mentioned.

In addition, as another embodiment of the detection method, when the mutation site of the gene is known, for example, the following methods may be employed:
the double-dye probe method (so-called TaqMan (registered trademark) probe method) where an oligonucleotide probe labeled with a reporter fluorescent dye and a quencher fluorescent dye, having a base sequence complementary to the base sequence comprising the mutation site in the gene region of the constituent protein, is hybridized with the above DNA sample used as a template, and the base sequence comprising the mutation site in the gene region of the constituent protein is amplified, and the fluorescence emitted by the reporter fluorescent dye is detected;
the HRM (high-resolution melting) method where in a reaction system comprising an intercalator that emits fluorescence when inserted between DNA double strands, the above DNA sample is used as a template to amplify and detect the base sequence comprising the mutation site in the gene region of the constituent protein; and
the DNA array method that uses a substrate formed by fixing the DNA comprising the mutation site in the gene region of the constituent protein, and the oligonucleotide probe hybridizing with the above DNA.

Furthermore, if the amino acid mutation site in the constituent protein is known, for example, one may employ a method where a protein sample is prepared from the target lactic acid bacteria, and a molecule that specifically binds to the amino acid mutation site (for instance, an antibody) is used to detect the mutation site; or a method such as peptide mass fingerprinting (PMF) or protein sequencers (Edman degradation method). For example, in the detection method using the antibody, an antigen-antibody reaction is performed using the antibody against the protein sample, to detect the constituent protein having the amino acid mutation site. If the antibody is labeled, it is possible to directly detect the target constituent protein, but if it is not labeled, furthermore, a labeled molecule that recognizes the antibody (such as secondary antibody or protein A) may be employed, allowing for indirect detection. Examples of such method include immunohistochemistry (immunostaining) method, Western blotting method, ELISA method, flow cytometry, imaging cytometry, radioimmunoassay, immunoprecipitation method, and analytical methods using antibody arrays. Note that there are no particular limitations on the method for preparing a protein sample from lactic acid bacteria, and a known method or a method similar thereto can be appropriately employed.

### [Decreased Expression of ATP Synthase Complex]

Decreased expression of the ATP synthase complex typically means that its expression level is less compared to the control. In the present invention, the "decreased expression of the ATP synthase complex" in lactic acid bacterium is synonymous with the decreased expression of at least one of the constituent proteins, and the method for verifying the decreased expression of the ATP synthase complex includes detecting the expression level of at least one of the constituent proteins at the transcription or translation level and comparing it with the control.

Among these, in the lactic acid bacterium according to the present invention, the decreased expression of the constituent protein (that is, function inhibition of the ATP synthase complex) is preferably a decreased expression of the A subunit protein and/or ε subunit protein, and more preferably a decreased expression of the ε subunit protein. More specifically, such a decreased expression is more preferably a decreased expression of (a) a protein composed of an amino acid sequence set forth in SEQ ID NO: 2 or SEQ ID NO: 16 (preferably SEQ ID NO: 16).

Furthermore, since in the natural world, the amino acid sequence encoded by a base sequence can mutate due to the mutation of that sequence, in the lactic acid bacterium according to the present invention, the decreased expression of the constituent protein may be a decreased expression of (b) a protein composed of an amino acid sequence in which one or more amino acids are substituted, deleted, inserted, and/or added in the amino acid sequence set forth in SEQ ID NO: 2 or SEQ ID NO: 16 (preferably SEQ ID NO: 16). In this case, one or more amino acid residues preferably means 1 or more and 10 or less amino acid residues, more preferably 1 or more and 5 or less, further preferably 1 or more and 3 or less, and most preferably 2 or less.

Moreover, with the current level of technology, those skilled in the art can, for example, upon obtaining the amino acid sequence set forth in SEQ ID NO: 2 or SEQ ID NO: 16, acquire genes encoding similar amino acid sequences, based on the nucleotide sequence encoding the same, using techniques such as hybridization (Southern, E. M., J. Mol. Biol., 98, 1975, p. 503-517) or polymerase chain reaction (PCR) (Saiki, R. K. et al., Science, 230, 1985, p. 1350-1354, Saiki, R. K et al., Science, 239, 1988, p. 487-491).

Therefore, as the lactic acid bacterium of the present invention, the decreased expression of the constituent protein mentioned above may be a decreased expression of (c) a protein composed of an amino acid sequence having 80% or more identity with the amino acid sequence set forth in SEQ ID NO: 2 or SEQ ID NO: 16 (preferably SEQ ID NO: 16). In this case, the identity of amino acid sequences can be determined, for example, using the BLASTP program (Altschul et al., J. Mol. Biol., 215, 1990, p. 403-410). Furthermore, it suffices that the identity with the amino acid sequence set forth in SEQ ID NO: 2 or SEQ ID NO: 16 is 80% or more, but preferably, it is 85% or more, 90% or more, or 95% or more (for example, 96% or more, 97% or more, 98% or more, or 99% or more).

The proteins (b) and (c) whose expression levels are to be detected need to be proteins without function inhibition. The constituent protein with decreased expression in the lactic acid bacterium of the present invention may be at least one of the above (a) and (b), but the above (a) is preferable.

### <Expression Level of ATP Synthase Complex Constituent Protein at Transcription Level>

In the method for detecting the expression level of the constituent proteins at the transcription level, for example, first, RNA is extracted from the target lactic acid bacteria, and reverse transcription from RNA is performed to prepare cDNA samples. There are no particular limitations on the method for extracting RNA from the lactic acid bacteria and the method for preparing cDNA samples, and for each of them, any known method or a method similar thereto can be appropriately employed. Next, using the aforementioned cDNA samples as a template, an amplification reaction using oligonucleotide primers or a hybridization reaction using an oligonucleotide probe is performed to detect its amplification products or hybrid products, and the quantity thereof can be quantified as needed, to determine the expression level at the transcription level. Examples of such methods include RT-PCR method, Northern blot method, dot blot method, DNA array method, in situ hybridization method, RNase protection assay method, and mRNA-seq. Those skilled in the art can design oligonucleotide primers and oligonucleotide probes suitable for each method in a usual manner, based on the typical sequences of amino acids of each constituent protein and the base sequences encoding them, which can be obtained from public databases (such as GenBank) (more specifically, for example, amino acid sequences and base sequences presented in Table 1).

If the detected expression level at the transcription level is reduced in comparison with the control, it can be determined that the expression level of the concerned constituent protein is reduced, that is, the ATP synthase complex is functionally inhibited in the lactic acid bacteria. The criteria for this determination are not particularly limited, but, for example, if the expression level is 4 SD or less, 3 SD or less, or 2 SD or less of the average expression level in the control, it may be determined that the expression level of the ATP synthase complex is reduced in the lactic acid bacteria.

As a control at this time, a cDNA sample prepared in the same way from lactic acid bacteria known to have no decreased expression in each constituent protein can be mentioned, and more specifically, for example, a cDNA sample prepared in the same way from OLS4496 strain can be mentioned.

### <Expression Level of ATP Synthase Complex Constituent Protein at Translation Level>

In the method for detecting the expression level of the constituent proteins at the translation level, for example, first, protein samples are prepared from the target lactic acid bacteria. There are no particular limitations on the method for preparing a protein sample from lactic acid bacteria, and a known method or a method similar thereto can be appropriately employed. Next, an antibody specific to the constituent proteins can be used to perform an antigen-antibody reaction, detect the constituent proteins, and quantify their amounts if necessary, determining the expression level at the translation level. Examples of such method for detecting proteins using antibodies include immunohistochemistry (immunostaining) method, Western blotting method, ELISA method, flow cytometry, imaging cytometry, radioimmunoassay, immunoprecipitation method, and analytical methods using antibody arrays.

In the method for detecting the expression level of constituent proteins at the translation level, detection of the constituent proteins can also be performed using mass spectrometry (MS). As a detection method using such mass spectrometry, for example, a method can be mentioned which involves labeling the proteins comprised in the protein samples, fractionating the labeled proteins, subjecting the fractionated proteins to mass spectrometry, identifying the constituent proteins from the mass spectrometric values, and quantifying them as necessary. For the aforementioned labeling, it is possible to use isotope-labeled reagents known in the art, and suitable labeling reagents can be obtained commercially. Also, fractionation can be performed by a method known in the art, for example, using commercially available ion exchange columns or the like.

Furthermore, it is known in the art that decreased expression of genes is due to factors such as excessive methylation of the promoter. Therefore, in detecting the expression level of the constituent proteins at the translation level, it is conceivable to detect the methylation of the gene promoter of the constituent proteins as an indicator. For the detection of promoter methylation, for example, known methods can be utilized such as a method that directly detects changes in the base sequence after bisulfite treatment which has the activity to convert methylated cytosine to uracil by base sequence determination, and a method that indirectly detects using a restriction endonuclease that can recognize (cut) the base sequence before bisulfite treatment but cannot recognize (cut) the base sequence after bisulfite treatment.

If the detected expression level at the translation level is reduced in comparison with the control, it can be determined that the expression level of the concerned constituent protein is reduced, that is, the ATP synthase complex is functionally inhibited in the lactic acid bacteria. The criteria for this determination are not particularly limited, but, for example, if the expression level is 4 SD or less, 3 SD or less, or 2 SD or less of the average expression level in the control, it may be determined that the expression level of the ATP synthase complex is reduced in the lactic acid bacteria.

As a control at this time, a protein sample prepared in the same way from lactic acid bacteria known to have no decreased expression in each constituent protein can be mentioned, and more specifically, for example, a protein sample prepared in the same way from OLS4496 strain can be mentioned.

In the present invention, as a method for confirming the "function inhibition of the ATP synthase complex" of the lactic acid bacterium (bacteria), any one of the above determination methods can be employed, and two or more thereof may be combined. Among these, from the viewpoints of accuracy and convenience, it is preferable to employ a method for determining the function inhibition of the ATP synthase complex by detecting a loss-of-function mutation in the genes of the constituent proteins in the target lactic acid bacteria.

### (Acid Production and Viable Bacterial Count during Low-Temperature Storage)

According to the lactic acid bacterium of the present invention, in the fermented milk obtained using such lactic acid bacterium, it becomes possible to reduce the amount of acid production during low-temperature storage, suppress pH decrease, and sufficiently maintain the viable bacterial count of the lactic acid bacteria even during the low-temperature storage. Therefore, the lactic acid bacterium of the present invention preferably satisfy the following conditions:
when cultured until the pH of the culture solution becomes 4.7 or less in a 10% skimmed milk powder medium at 43°C, followed by storage at 10°C for 10 days,
(A1) the viable bacterial count in the culture solution after the storage becomes 1 × 10⁸ cfu/g or more, and
(A2) the pH of the culture solution after the storage becomes 4.2 or higher.

Further, the lactic acid bacterium of the present invention more preferably satisfy the following conditions:
(A3) the time until the pH of the culture solution becomes 4.7 or lower during the culture is 7 hours or less from the start of the culture, and/or
(A4) the pH of the culture solution after 20 hours from the start of the culture is 4.1 or higher.

### [Skimmed Milk Powder Medium]

In conditions (A1) to (A4), "10% skimmed milk powder medium" refers to a medium comprising 10% by mass of skimmed milk powder. The skimmed milk powder according to the present invention refers to defatted animal milk (preferably cow's milk), formed into skim milk and dried into a powdered form, and examples thereof include those having 95.0% or more of milk solids and 5.0% or less of moisture content, as stipulated in Japan's "Ministerial Ordinance on Milk and Milk Products Concerning Compositional Standards, etc. (hereinafter sometimes referred to as 'Ministerial Ordinance on Milk and Milk Products')." The composition of such skimmed milk powder includes, for example, the composition described in the "Standards Tables of Food Composition in Japan -2015-(Seventh Revised Edition)," that is, per 100 g, 300 to 400 (preferably 359) kcal of energy, 3 to 5 (preferably 3.8) g of moisture, 30 to 40 (preferably 34.0) g of protein, 0.2 to 2 (preferably 1.0) g of fat, 45 to 60 (preferably 53.3) g of carbohydrates (including lactose), 900 to 2000 (preferably 1100) mg of calcium, 3 to 10 (preferably 6) µg of vitamin A, 0.5 to 2 (preferably 1.60) mg of vitamin B₂, 10 to 30 (preferably 25) mg of cholesterol, and 0.5 to 2 (preferably 1.4) g of equivalent salt content. The pH of such 10% skimmed milk powder medium is typically within the range of 6 to 7.

For the 10% skimmed milk powder medium according to conditions (A1) to (A4), it is permissible to include additional components besides skimmed milk powder; however, even in such cases, it is preferable that the content of the additional components be 0.1% by mass or less, and more preferably 0.01% by mass or less, with the medium composed of 10% by mass of skimmed milk powder and 90% by mass of water (preferably potable water) being particularly preferable. As the aforementioned additional components, for example, yeast extract and peptides are mentioned, but especially for the 10% skimmed milk powder medium according to the above conditions, it is preferable that the yeast extract is substantially absent. Note that, in the present invention, "substantially absent" refers to being 0.001% by mass or less with respect to the total mass. The lactic acid bacterium of the present invention can fulfill the above conditions even in a medium not comprising the yeast extract.

For the 10% skimmed milk powder medium according to conditions (A1) to (A4), it is preferable that total sterilization treatment or partial sterilization treatment has been applied. Although the conditions therefore are not specifically limited, conditions for the total sterilization treatment can include temperatures of 70 to 130°C for 1 minute to 1 hour, and conditions for the partial sterilization treatment can include temperatures of 75 to 125°C for 10 to 30 minutes. Additionally, for the 10% skimmed milk powder medium, it may have the same composition and/or the same total sterilization treatment or partial sterilization treatment as the first 10% skimmed milk powder medium and the second 10% skimmed milk powder medium below, or it may have different compositions and/or treatments. Furthermore, when conducting the activation culture below, the second 10% skimmed milk powder medium may comprise a part or all of the culture solution after the first culture.

### [Activation Culture (First Culture)]

In conditions (A1) to (A4), when lactic acid bacteria have been stored at low temperatures or frozen, it is preferable to first perform activation culture on the lactic acid bacteria. As such activation culture (hereinafter sometimes referred to as "first culture"), a 10% skimmed milk powder medium (hereinafter, the medium for the first culture is sometimes referred to as "first 10% skimmed milk powder medium") is used, and culture is preferably conducted at 37°C, anaerobically, for 16 to 18 hours (preferably 16 hours). In this case, the amount of lactic acid bacteria subjected to the first culture is preferably an amount that results in a viable bacterial count of 1 × 10² to 2 × 10⁸ cfu/g, and more preferably 1 × 10⁴ to 1 × 10⁶ cfu/g, in the first 10% skimmed milk powder medium.

Furthermore, in the present invention, measurement of viable bacterial count can be carried out, for example, by culturing a diluted solution of lactic acid bacteria-comprising liquid (for example, in the case of the first culture, the culture solution of the first culture (lactic acid bacteria and first 10% skimmed milk powder medium)), suitably diluted, on a suitable agar medium, such as BCP added plate count agar medium defined by the Ministerial Ordinance on Milk and Milk Products, and counting the number of colonies that appear. The value of viable bacterial count (cfu/g) in this case is the viable bacterial count in the lactic acid bacteria-comprising liquid (g), which hence can be used to calculate the viable bacterial count in the culture solution of the first culture, for instance.

As for the anaerobic culture method, although not particularly limited, known methods for anaerobic culture of lactic acid bacteria conventionally exist, such as stationary culture in the presence of an oxygen concentration regulator (such as AnaeroPack Kenki, manufactured by Mitsubishi Gas Chemical Company). In the present invention, it is also acceptable to re-inoculate the culture solution after the aforementioned culture into the first skimmed milk powder medium, and perform the first culture multiple times (for example, up to 2 times).

### [Culture (Second Culture)]

In conditions (A1) to (A4), culture is performed using lactic acid bacteria or the culture solution after the first culture (that is, the activated lactic acid bacteria-comprising culture solution) (hereinafter sometimes referred to as "second culture"). In the second culture, culture is performed at 43°C in a 10% skimmed milk powder medium (hereinafter, the medium for the second culture is sometimes referred to as "second 10% skimmed milk powder medium"). In this case, the amount of lactic acid bacteria subjected to the second culture is preferably an amount that results in a viable bacterial count of 2 × 10⁶ to 5 × 10⁷ cfu/g, more preferably 4 × 10⁶ to 4 × 10⁷ cfu/g, and more preferably 1 × 10⁷ to 2 × 10⁷ cfu/g, in the second 10% skimmed milk powder medium. The culture method in the second culture is not particularly limited and can include fermentation methods for fermented milk using conventional lactic acid bacteria, such as stationary culture, as appropriate.

In the second culture, when fermentation occurs due to the metabolism of lactic acid bacteria producing lactic acid from lactose comprised in the second 10% skimmed milk powder medium, the pH of the culture solution decreases due to the produced lactic acid. In the second culture according to the above conditions, the completion of this fermentation is determined by the pH of the culture solution (that is, the pH of the second 10% skimmed milk powder medium comprising lactic acid bacteria) becoming 4.7 or lower (more preferably 4.6 or lower).

Condition (A3) requires that the time until completion of the fermentation (that is, when pH reaches 4.7) is 7 hours or less from the start of the second culture, particularly preferably 6 hours or less, and further preferably 5 hours or less. For lactic acid bacteria that exceed the upper limit in the time until completion of the fermentation, it is not preferable for production efficiency because it takes time to produce when used in the production of fermented milk, and the viable bacterial count in the fermented milk decreases upon low-temperature storage. While there is no particular limitation on the lower limit of the time until completion of the fermentation, for instance, it is preferable to be 1 hour or more, and more preferable to be 2 hours or more.

When the second culture continues for 20 hours or more, typically, the pH of the culture solution further decreases due to the continued production of lactic acid and the following increase in this produced lactic acid. In contrast, under condition (A4), it is necessary for the pH of the culture solution 20 hours after the start of the second culture to be 4.1 or higher, and it is particularly preferable for the pH of the culture solution 24 hours after the start of culture to be 4.1 or higher. Moreover, there is no particular limitation on the upper limit of the time from the start of the second culture to maintain a pH of 4.1 or higher, but for example, it is preferable to be 30 hours or less. The present inventors have found that lactic acid bacteria that can maintain a pH of 4.1 or higher even after long-term culture at 43°C can also suppress the decrease in pH during low-temperature (such as 10°C) storage of the resulting fermented milk. Note that even lactic acid bacteria with low fermentation capability may maintain a pH of 4.1 or higher after long-term culture at 43°C, but in this case, condition (A1) and/or condition (A3) is not satisfied.

### [Storage]

In conditions (A1) and (A2), the culture solution after the fermentation is completed (that is, after reaching pH 4.7) is promptly cooled and stored. While the conditions for storage are not particularly limited, methods known as conventional fermented milk storage methods can be mentioned, such as a method involving sealing (preferably airtight sealing) the culture solution in a container and allowing it to stand.

As for the storage temperature according to conditions (A1) and (A2), it is necessary for it to be a low temperature, more specifically, it needs to be 10°C. Additionally, for the storage period based on these conditions, it is necessary for each independently to be 10 days or more, particularly preferably 16 days or more, more preferably 20 days or more, and further preferably 35 days or more. In the lactic acid bacterium of the present invention, even when stored for a long period under such low-temperature conditions, the amount of acid production is sufficiently low, and the viable bacterial count is adequately maintained.

Since lactic acid bacteria can typically produce the aforementioned lactic acid even at low temperatures, when the culture solution after completion of fermentation is stored for a long time as mentioned above, even under low-temperature conditions, the pH of the culture solution further decreases with an increase in the amount of the lactic acid produced. In contrast, in condition (A2), it is necessary for the pH of the culture solution (that is, the pH of the second 10% skimmed milk powder medium comprising lactic acid bacteria) after being stored for 10 days at 10°C to be 4.2 or higher (preferably 4.3 or higher, more preferably 4.4 or higher), and it is particularly preferable for the pH of the culture solution after being stored for 16 days at 10°C to be 4.2 or higher (preferably 4.3 or higher, more preferably 4.4 or higher), and it is further preferable for the pH of the culture solution after being stored for 35 days at 10°C to be 4.2 or higher (preferably 4.3 or higher, more preferably 4.4 or higher).

Among the conventional lactic acid bacteria, there are lactic acid bacteria known for their sensitivity to low temperatures, in which case even when stored at low temperatures for extended periods, the pH of the culture solution may not decrease. However, the present inventors have newly discovered that conventionally known low-temperature-sensitive lactic acid bacteria, such as those described in PTL 2, may face the problem of a long time required to complete fermentation or a reduced viable bacterial count due to low-temperature storage. Regarding this, the present inventors speculate that in conventionally known low-temperature-sensitive lactic acid bacteria, such as the bacteria described in PTL 3, if the pH decreases, apoptosis occurs where H⁺ is not discharged outside the bacterial body, leading to a decrease in the pH in the bacterial body and inhibiting growth. In contrast, the present inventors have found lactic acid bacteria that can suppress the decrease in pH during low-temperature storage and can sufficiently maintain viable bacterial count during such low-temperature storage, that is, lactic acid bacteria that satisfy condition (A1). In condition (A1), it is necessary for the viable bacterial count in the culture solution after being stored for 10 days at 10°C to be 1 × 10⁸ cfu/g or higher, it is particularly preferable for the viable bacterial count in the culture solution after being stored for 16 days at 10°C to be 1 × 10⁸ cfu/g or higher, it is more preferable for the viable bacterial count in the culture solution after being stored for 20 days at 10°C to be 1 × 10⁸ cfu/g or higher, and it is further preferable for the viable bacterial count in the culture solution after being stored for 35 days at 10°C to be 1 × 10⁸ cfu/g or higher. Note that the conventionally known low-temperature-sensitive lactic acid bacteria at least do not satisfy condition (A1) and/or condition (A3).

The lactic acid bacterium of the present invention may be a single strain or a combination of two or more strains. Specifically, more specific examples of lactic acid bacterium of the present invention include Streptococcus thermophilus OLS4801 (hereinafter sometimes referred to as "OLS4801 strain"), specified by accession number NITE BP-03504, Streptococcus thermophilus OLS4802 (hereinafter sometimes to as "OLS4802 strain"), specified by accession number NITE BP-03505, Streptococcus thermophilus OLS4803 (hereinafter sometimes to as "OLS4803 strain"), specified by accession number NITE BP-03506, and Streptococcus thermophilus OLS4824 (hereinafter sometimes to as "OLS4824 strain"), specified by accession number NITE BP-03508. All of these exemplified lactic acid bacteria are S. thermophilus, and possess the prtS gene.

The OLS4801 strain and OLS4802 strain have a loss-of-function mutation in the ε subunit gene as a function inhibition of the ATP synthase complex, where the glycine (Gly) corresponding to the 30th glycine in the amino acid sequence set forth in SEQ ID NO: 16 is substituted with alanine (Ala) (G30A). In addition, the OLS4803 strain has a loss-of-function mutation in the ε subunit gene as a function inhibition of the ATP synthase complex, where the base corresponding to the 258th A in the base sequence set forth in SEQ ID NO: 15 is deleted, and a frameshift has caused substitution of the glutamic acid (Glu) corresponding to the 86th glutamic acid in the amino acid sequence set forth in SEQ ID NO: 16 and subsequent amino acids thereof (Q86FS). Furthermore, the OLS4824 strain has a loss-of-function mutation in the A subunit gene as a function inhibition of the ATP synthase complex, where the valine (Val) corresponding to the first valine of the amino acid sequence set forth in SEQ ID NO: 2 is substituted with alanine (Ala) (V1A).

Each strain has been deposited, that is, OL4801 strain: (1) identification label: Streptococcus thermophilus OL4801, (2) accession number: NITE BP-03504, (3) accession date: August 4, 2021, OL4802 strain: (1) identification label: Streptococcus thermophilus OL4802, (2) accession number: NITE BP-03505, (3) accession date: August 4, 2021, OL4803 strain: (1) identification label: Streptococcus thermophilus OL4803, (2) accession number: NITE BP-03506, (3) accession date: August 4, 2021, and OL4824 strain: (1) identification label: Streptococcus thermophilus OL4824, (2) accession number: NITE BP-03508, (3) accession date: August 4, 2021, at (4) depositary institution: National Institute of Technology and Evaluation NITE Patent Microorganisms Depositary (postal code 292-0818, Room 122, 2-5-8 Kazusa Kamatari, Kisarazu City, Chiba Prefecture). Note that the S. thermophilus identified by these accession numbers may be a subcultured strain of the same strain, or may be an artificial mutant strain, a natural mutant strain, a genetically modified strain, a derivative strain, or the like of the same strain or a subcultured strain thereof, as long as the effects of the present invention are not impaired.

Furthermore, the lactic acid bacterium of the present invention may be lactic acid bacterium prepared by artificially introducing a function inhibition of the aforementioned ATP synthase complex appropriately using known or naturally occurring lactic acid bacterium (preferably S. thermophilus, and more preferably S. thermophilus possessing the prtS gene). As methods for artificially introducing a function inhibition of the ATP synthase complex into lactic acid bacterium, it is possible to employ appropriate conventionally known methods according to the mode of the aforementioned function inhibition, and examples thereof include introducing loss-of-function mutations into the genes of each constituent protein by site-specific mutation introduction method, homologous recombination method, or gene targeting method (knockout or knock-in); inducing mutations (inducing loss-of-function mutations) in the genes of each constituent protein by radiation treatment with X-rays, alpha rays, beta rays, gamma rays, ion beams, or the like; inducing mutations (inducing loss-of-function mutations) in the genes of each constituent protein by ultraviolet radiation; inducing mutations (inducing loss-of-function mutations) in the genes of each constituent protein with chemicals such as ethyl methanesulfonate (EMS); and methods such as decreasing the expression level by methylation of the gene promoters of each constituent protein can be mentioned.

In addition, the lactic acid bacterium of the present invention may further possess mutations other than the above-described loss-of-function mutation in the genes of the constituent proteins.

### <Method for Screening Lactic Acid Bacterium>

In lactic acid bacterium with function inhibition of the ATP synthase complex, during low-temperature storage, the amount of acid production is low and the viable bacterial count is not reduced even by the low-temperature storage. Therefore, using the function inhibition of the ATP synthase complex as an indicator, it becomes possible to screen for suitable lactic acid bacterium for producing fermented milk in which decrease in pH during low-temperature storage is suppressed and the viable bacterial count of lactic acid bacteria is sufficiently maintained. Accordingly, the present invention also provides a method for screening lactic acid bacterium including a selection step of selecting lactic acid bacterium with low acid production using the function inhibition of the ATP synthase complex as an indicator. In the present invention, "lactic acid bacterium (bacteria) with low acid production" refers to lactic acid bacterium that, when used, can suppress the decrease in pH during low-temperature storage and maintain viable bacterial counts in the resulting fermented milk. More specifically, examples include lactic acid bacterium that satisfy the above conditions (A1) and (A2), and preferably satisfy the above conditions (A3) and/or (A4).

In the aforementioned selection step, in the lactic acid bacterium of the present invention, it is determined whether the ATP synthase complex is functionally inhibited by at least one of the determination methods listed in Function Inhibition of ATP Synthase Complex (that is, Direct Activity Reduction of ATP Synthase Complex, Genetic Mutation of ATP Synthase Complex Constituent Protein, and Decreased Expression of ATP Synthase Complex), and the lactic acid bacterium confirmed to have a function inhibition of the ATP synthase complex are selected as the lactic acid bacterium with low acid production. The determination method is as mentioned above, including preferable embodiments thereof.

Moreover, the screening method of the present invention further includes a step of performing culture and storage according to each condition mentioned in Second Culture and, as needed, First Culture, as well as Storage for the lactic acid bacterium according to the present invention, and determining the lactic acid bacterium that satisfy the conditions (A1) and (A2) and, as needed, conditions (A3) and/or (A4), as the lactic acid bacterium with low acid production, and selecting them. The above conditions are as described in the lactic acid bacterium of the present invention, including their preferable embodiments.

### <Lactic Acid Bacterium Composition>

The term "lactic acid bacterium composition" refers to a composition comprising lactic acid bacterium, without particular limitations, and as the lactic acid bacterium composition of the present invention, it suffices to comprise at least the lactic acid bacterium of the present invention (including lactic acid bacterium selected by the method for screening lactic acid bacterium of the present invention), and may be composed solely of the lactic acid bacterium (bacteria) of the present invention (including a combination of two or more types of lactic acid bacteria of the present invention). The lactic acid bacterium composition of the present invention encompasses, for example, the following lactic acid bacterium starter and fermented milk. As the lactic acid bacterium composition of the present invention, it is also acceptable to further comprise additional components other than the lactic acid bacterium of the present invention, and these additional components are not particularly limited and may include solvents such as water; cultures such as the supernatant of the culture solution after the lactic acid bacteria have finished culturing, and culture medium components; concentrated, diluted, dried, frozen forms of the culture, and the like, and these can be singular or in combinations of two or more.

### (Lactic Acid Bacterium Starter)

The lactic acid bacterium starter of the present invention, which comprises at least the lactic acid bacterium of the present invention, can be suitably used for the method for producing fermented milk of the present invention described later. As such lactic acid bacterium starter of the present invention, it may be composed solely of the lactic acid bacterium (bacteria) of the present invention, or it may further comprise the aforementioned additional components, and additional lactic acid bacteria or yeast.

Regarding additional lactic acid bacteria and yeast, known lactic acid bacteria and yeast that have conventionally been included in fermented milk are mentioned. Examples of additional lactic acid bacteria include lactic acid bacteria other than the lactic acid bacteria of the present invention, and these can be singular or in combinations of two or more. Among these, if the lactic acid bacterium of the present invention is lactic acid bacterium of the genus Streptococcus, as a lactic acid bacterium starter of the present invention, it is preferable to further comprise lactic acid bacteria of the genus Lactobacillus other than the lactic acid bacteria of the present invention, and more preferably, to comprise lactic acid bacteria belonging to Lactobacillus delbrueckii subsp. bulgaricus. In addition, the lactic acid bacterium starter of the present invention also preferably comprises lactic acid bacteria of the genus Streptococcus, which is the lactic acid bacterium of the present invention, and lactic acid bacteria of the genus Lactobacillus, which is the lactic acid bacterium of the present invention.

The lactic acid bacterium starter of the present invention may be in a liquid state or in a solid state such as a frozen state or a dry powder, and may further comprise additional components. Examples of the further additional components that can be comprised in the lactic acid bacterium starter include fermentation promoting substances (such as formic acid and nucleic acids); protective agents (saccharides); and medium components (such as milk and whey), and may be one of these or a combination of two or more thereof.

The content of the lactic acid bacteria of the present invention in the lactic acid bacterium starter of the present invention is not particularly limited, but preferably 0.01 to 100% by mass, and more preferably 0.1 to 90% by mass. Also, in terms of the viable bacterial count, it is preferable to be 1 × 10⁷ cfu/g or more, and more preferably 1 × 10⁷ to 1 × 10¹¹ cfu/g. Furthermore, in the case of further comprising additional lactic acid bacteria, consider the ratio of the content of the lactic acid bacteria of the present invention (preferably, lactic acid bacteria of the genus Streptococcus) in the lactic acid bacteria starter and the content of the additional lactic acid bacteria (preferably, lactic acid bacteria of the genus Lactobacillus other than the lactic acid bacteria of the present invention). Also, in the case of comprising lactic acid bacteria of the genus Streptococcus, which is the lactic acid bacterium of the present invention, and lactic acid bacteria of the genus Lactobacillus, which is the lactic acid bacterium of the present invention, consider the ratio of the content of the lactic acid bacteria of the genus Streptococcus and the content of the lactic acid bacteria of the genus Lactobacillus in the lactic acid bacteria starter. These ratios are each independently preferably 1:0.1 to 1:100, more preferably 1:1 to 1:10, in a bacterial count ratio (in terms of viable bacterial count, hereinafter the same).

The lactic acid bacterium starter of the present invention may, for example, be combined with a composition comprising the additional lactic acid bacteria (second lactic acid bacteria composition) to form a lactic acid bacterium starter kit including the lactic acid bacterium starter of the present invention and the second lactic acid bacterium composition. Additionally, it may be, for example, in the form of a lactic acid bacterium starter kit including additives for producing fermented milk (such as fermentation promoting substances and protective agents described above), containers, and usage instructions for the lactic acid bacterium starter.

### (Fermented Milk)

The fermented milk of the present invention comprises at least the lactic acid bacterium of the present invention, and for example, can be obtained by the method for producing fermented milk of the present invention described later. In the fermented milk of the present invention, the decrease in pH during low-temperature storage is suppressed, and the viable bacterial count comprised even by the low-temperature storage is sufficiently maintained.

The fermented milk of the present invention is not particularly limited, and examples thereof include fermented milk that satisfies the standards for "fermented milk" according to the Ministerial Ordinance on Milk and Milk Products (more specifically, the content of milk solids-not-fat is 8.0% or more, and the lactic acid bacteria count or yeast count (preferably the lactic acid bacteria count (more preferably the count of the lactic acid bacteria of the present invention), hereinafter the same) is 10 million/mL or more). In addition, the fermented milk of the present invention also includes those that satisfy the standards for "milk products and fermented milk drinks" (more specifically, the content of milk solids-not-fat is 3.0% or more, and the lactic acid bacteria count or yeast count is 10 million/mL or more); those that satisfy the standards for "fermented milk drinks" (more specifically, the content of milk solids-not-fat is 3.0% or more, and the lactic acid bacteria count or yeast count is 1 million/mL or more), according to the above Ministerial Ordinance on Milk and Milk Products. Note that the milk solids-not-fat refers to the remaining components (mainly such as proteins, lactose, and minerals) obtained by subtracting the fat content from the total milk solids, and the lactic acid bacteria and yeast count are viable bacterial counts measured using the test method stipulated in the Ministerial Ordinance on Milk and Milk Products and the method using the BCP added plate count agar medium, and in the case of fermented milk that has undergone partial sterilization, they are in terms of the viable bacterial counts measured by the aforementioned test method before the partial sterilization.

The fermented milk of the present invention may be a fermented product after the fermentation step of the method for producing fermented milk described later or may be obtained by partially sterilizing the fermented product (such as pulverization or heat treatment), or may be obtained by, for example, concentrating, diluting, drying, or freezing them. Note that in the present invention, when the fermented milk is partially sterilized, the lactic acid bacteria count in the fermented milk is in terms of viable bacterial count. The lactic acid bacteria comprised in the fermented milk of the present invention include not only viable bacteria but also dead bacteria, as well as disrupted products and heat-treated products of lactic acid bacteria, and concentrates, dilutions, dried products, and frozen products thereof. The fermented milk of the present invention preferably comprises at least viable bacteria of lactic acid bacteria, and more preferably comprises the lactic acid bacteria of the present invention as viable bacteria.

The fermented milk of the present invention comprises components derived from the lactic acid bacterium (bacteria) of the present invention and the raw material milk (preferably formula milk) described later. Moreover, within the scope not inhibiting the effects of the present invention, it may further comprise additional components listed in Lactic Acid Bacterium Starter mentioned above, as well as additional lactic acid bacteria and yeast. In addition, it may comprise various components that can be comprised in food and drink. Further components that can be comprised in fermented milk are not particularly limited, and examples thereof include saccharides, sugar alcohols, minerals, vitamins, proteins, peptides, amino acids, organic acids, pH adjusters, starches and modified starches, dietary fibers, fruits and vegetables and processed products thereof, animal and plant crude drug extracts, naturally-derived polymers (such as collagen, hyaluronic acid, and chondroitin), oils and/or fats, thickeners, emulsifiers, solvents, surfactants, gelling agents, stabilizers, buffers, suspending agents, thickening agents, excipients, disintegrators, binders, flow agents, preservatives, colorants, fragrances, corrigents, and sweeteners, and may be one of these or a combination of two or more thereof.

Such fermented milk is preferably yoghurt, cheese, fermented cream, fermented butter, and the like, and particularly preferably yogurt. Specific examples of the yogurt include set type yogurt (solid fermented milk) such as plain yogurt, soft-type yogurt (pasty fermented milk), and drink type yogurt (liquid fermented milk), and frozen yogurt using these ingredients may also be used. In addition, the fermented milk of the present invention can also be used as an ingredient for fermented food such as cheese, fermented cream, fermented butter, and kefir. Furthermore, the fermented milk of the present invention can also be used as a lactic acid bacterium starter in the method for producing fermented milk, provided that it has not been subjected to partial sterilization.

### <Method for Producing Fermented Milk>

The method for producing fermented milk of the present invention includes a fermentation step of adding the lactic acid bacterium of the present invention (including lactic acid bacterium selected by the method for screening lactic acid bacterium of the present invention) or the lactic acid bacterium composition of the present invention to a formula milk comprising raw material milk, followed by fermentation to thereby obtain fermented milk. Note that in the case of using the lactic acid bacterium selected by the method for screening lactic acid bacterium of the present invention, the method for producing fermented milk of the present invention may include the selection step, and the selection step may be only the initial step. According to the production method of the present invention, by using the lactic acid bacterium of the present invention to ferment the raw material milk, it becomes possible to produce fermented milk in which the decrease in pH during low-temperature storage is suppressed, and the viable bacterial count comprised even by the low-temperature storage is sufficiently maintained. Furthermore, the production method of the present invention also makes it possible to significantly shorten the fermentation time until completion.

### (Formula Milk)

The formula milk according to the present invention comprises raw material milk. The raw material milk preferably comprises lactose, and examples thereof include raw milk (such as milk of cow, water buffalo, sheep, goat, and the like), partially sterilized milk, whole milk, skim milk, whey, and processed products thereof (such as whole milk powder, full-fat concentrated milk, skimmed milk powder, skimmed concentrated milk, condensed milk, whey powder, buttermilk, butter, cream, cheese, whey protein concentrate (WPC), whey protein isolate (WPI), α-lactalbumin (α-La), and β-lactoglobulin (β-Lg)), and may be one of these or a mixture of two or more thereof.

The formula milk according to the present invention may be composed only of the above raw material milk, or may be an aqueous solution, diluted solution, or concentrated solution of the above raw material milk, or may further comprise additional components in addition to the above raw material milk, if necessary. Examples of the additional components include water; foods, food ingredients, or food additives such as soymilk, saccharides such as sugar, sweeteners, fragrances, fruit juices, fruit pulps, vitamins, minerals, oils and/or fats, ceramides, collagen, milk phospholipids, and polyphenols; stabilizers, thickeners, and gelling agents such as pectin, soy polysaccharides, CMC (carboxymethylcellulose), agar, gelatin, carrageenan, and gums, and may be one of these or a mixture of two or more thereof. The formula milk can be prepared by mixing the above components, while optionally with heating and/or optionally with homogenizing. In addition, as the formula milk, heat total sterilized or partial sterilized one can also be used.

### (Fermentation)

In the fermentation step of adding the lactic acid bacterium of the present invention or the lactic acid bacterium composition of the present invention to the aforementioned formula milk, followed by fermentation, conventionally known methods can be appropriately employed without particular limitations. Moreover, the amount of the lactic acid bacterium of the present invention or the lactic acid bacterium composition of the present invention to be added can be appropriately set according to the amount of lactic acid bacterium starter used in the conventionally known method for producing fermented milk, but is, for example, preferably 1 × 10⁷ to 5 × 10⁹ cfu/g, more preferably 1 × 10⁸ to 2 × 10⁹ cfu/g, in a viable bacterial count of the lactic acid bacteria of the present invention, based on the mass of the formula milk.

To the formula milk, in addition to the lactic acid bacterium of the present invention or the lactic acid bacterium composition of the present invention, the additional lactic acid bacteria and yeast may be added. Among these, if the lactic acid bacterium of the present invention is lactic acid bacterium of the genus Streptococcus, for the method for producing fermented milk of the present invention, it is preferable to further add lactic acid bacteria of the genus Lactobacillus as the additional lactic acid bacteria, and more preferably to further add lactic acid bacteria belonging to Lactobacillus delbrueckii subsp. bulgaricus. Furthermore, in the case of further adding additional lactic acid bacteria, consider the ratio of the amount of the lactic acid bacteria of the present invention (preferably lactic acid bacteria of the genus Streptococcus) added and the amount of the additional lactic acid bacteria (preferably lactic acid bacteria of the genus Lactobacillus) added. Also, in the case of using the lactic acid bacteria of the genus Streptococcus, which is the lactic acid bacterium of the present invention, and the lactic acid bacteria of the genus Lactobacillus, which is the lactic acid bacterium of the present invention, consider the ratio of the amount of the lactic acid bacteria of the genus Streptococcus added and the amount of the lactic acid bacteria of the genus Lactobacillus added. These ratios are each independently preferably 1:0.1 to 1:100, more preferably 1:1 to 1:10, in a bacterial count ratio (in terms of viable bacterial count, hereinafter the same).

The fermentation conditions are not particularly limited, and can be appropriately selected depending on the growth conditions of the lactic acid bacterium of the present invention to be added, the amount of the formula milk, and the like. For example, under aerobic or anaerobic conditions at a temperature of 35 to 45°C and more preferably at a temperature of 38 to 43°C, the mixture is allowed to stand or stirred (preferably stand) for usually 2 to 24 hours, more preferably 3 to 8 hours, and further preferably 4 to 6 hours until the pH of the formula milk added with the lactic acid bacterium of the present invention or the lactic acid bacterium composition of the present invention is 4.7 or less, more preferably 4.6 or less, and further preferably 4.0 to 4.5. According to the present invention, it is possible to sufficiently shorten the time required for fermentation. In addition, as the anaerobic conditions, for example, fermentation under nitrogen aeration conditions can be employed.

The fermented milk of the present invention can be obtained by the above fermentation. The fermented product after the fermentation step can be used as the fermented milk of the present invention as it is or by concentrating, diluting, drying, freezing, or the like as necessary. Also, the fermented milk of the present invention may be obtained by disrupting or heat-treating the lactic acid bacteria in the fermented product, or by concentrating, diluting, drying, freezing, or the like as necessary. Therefore, the method for producing fermented milk of the present invention may further include these steps (such as concentration step, dilution step, drying step, freezing step, disrupting step, and heat treatment step).

### [Examples]

Hereinafter, the present invention will be described in more detail based on the Examples and Comparative Examples, but the present invention is not limited to the following Examples. Note that in each of the Test Examples below, the expression "w/v%" indicates weight/volume (w/v: g/mL) percent unless otherwise specified.

### (Test Example 1)

(1) The following three strains of lactic acid bacteria were used:
   P2101201 strain (Comparative Example 1): S. thermophilus possessing the prtS gene. The bacterial strain is preserved by the Meiji Innovation Center of Meiji Co., Ltd. (postal code 192-0919, 1-29-1 Nanakuni, Hachioji City, Tokyo, Japan);
   OLS4801 strain (Example 1): S. thermophilus specified by accession number NITE BP-03504. It possesses the prtS gene;
   OLS4802 strain (Example 2): S. thermophilus specified by accession number NITE BP-03505. It possesses the prtS gene; and
   OLS4803 strain (Example 3): S. thermophilus specified by accession number NITE BP-03506. It possesses the prtS gene.
   In the test examples described in the present specification, the presence or absence of the prtS gene in each lactic acid bacterial strain was confirmed by the following method. Specifically, first, the prtS gene sequences of 5 strains of S. thermophilus with known genome sequences were obtained from the NCBI database, and the highly conserved sequences were used to prepare primers (Fprimer: SEQ ID NO: 17 and Rprimer: SEQ ID NO: 18). In addition, InstaGene Matrix (manufactured by BioRad) was used to extract genomic DNA from the M17 culture solution of each bacterial strain. 0.5 µL of extracted genomic DNA (template), 1 µL each of the prepared primers (5 µM), 0.1 µL of Phusion high fidelity DNA polymerase, 2 µL of 5 × HF buffer, 0.8 µL of 2.5 mM dNTP, and 4.6 µL of ultrapure water were mixed (total 10 µL), and PCR was performed under the following conditions: at 98°C for 30 seconds; 30 cycles of 98°C for 5 seconds, 63°C for 20 seconds, and 72°C for 20 seconds; and 72°C for 5 minutes; and allowed to stand at 4°C. The resulting PCR products were subjected to agarose gel electrophoresis, and bacterial strains confirmed to have a band at 684 bp were determined to have the prtS gene, and bacterial strains not confirmed to have the band were determined not to have the prtS gene.
(2) In a 10% skimmed milk powder medium that had been subjected to total sterilization at 121°C for 7 minutes (10 parts by mass of skimmed milk powder and 90 parts by mass of distilled water (10% by mass of skimmed milk powder), pH: 6 to 7, hereinafter the same), the four strains of lactic acid bacteria were individually subjected to stationary culture for 16 hours at 37°C under anaerobic conditions (using an oxygen concentration regulator (AnaeroPack Kenki, manufactured by Mitsubishi Gas Chemical Company), hereinafter the same) for activation. Note that the bacterial count in the culture solution after activation was 5 × 10⁸ to 1 × 10⁹ cfu/g. Subsequently, a 10% skimmed milk powder medium that had been subjected to partial sterilization at 75°C for 15 minutes was inoculated such that the mass of the culture solution was 2% by mass (viable bacterial count of lactic acid bacteria: 1 × 10⁷ to 2 × 10⁷ cfu/g, hereinafter the same), and subjected to stationary culture (fermentation) at 43°C, and the time (fermentation time) from the inoculation until the pH of the culture solution decreased to 4.7 was measured. Furthermore, the culture was terminated when the pH reached 4.7, and immediately it was cooled down to 10°C, sealed tightly to prevent the entry of air, and allowed to stand for 10 days, 17 days, 20 days, or 35 days to measure each pH (10°C pH) after storage. The results are shown in Table 2 below.

**[Table 2]**

| Lactic Acid Bacteria | Fermentation Time (Min) | 10°C pH | | | |
|---|---|---|---|---|---|
| | | 10 Days | 17 Days | 20 Days | 35 Days |
| P2101201 | 240 | 4.15 | 4.11 | - | 4.11 |
| OLS4801 | 245 | 4.44 | 4.42 | - | 4.41 |
| OLS4802 | 235 | 4.45 | 4.42 | - | 4.42 |
| OLS4803 | 235 | 4.57 | - | 4.52 | 4.51 |

As shown in Table 2, fermentation was completed within 5 hours for each of the lactic acid bacteria P2101201 strain, OLS4801 strain, OLS4802 strain, and OLS4803 strain (reaching a pH of 4.7). On the other hand, regarding the time course in pH with storage at 10°C, it was confirmed that the P2101201 strain was a high acid-producing lactic acid bacteria, as the pH dropped to 4.11 during a 17-day storage period. In contrast, it was confirmed that the OLS4801, OLS4802, and OLS4803 strains were low acid-producing lactic acid bacteria, as the pH did not fall below 4.4 even after 35 days of storage.

### (Test Example 2)

(1) The same strains of lactic acid bacteria as the four strains of lactic acid bacteria used in Test Example 1 were cultured separately in M17 culture solution (0.5w/v% lactose) at 37°C under anaerobic conditions for 16 hours, followed by centrifugation to harvest the bacteria, which were then washed with physiological saline, harvested again by centrifugation. The resultant was suspended in 100 µL of a solution (6.7% sucrose, 50 mM Tris-HCl, 1 mM EDTA (pH 8.0)). To the suspension, 20 µL of 10 mg/mL lysozyme and 5 µL of 10 U/uL mutanolysin were added, and the bacteria were lysed at 37°C for 10 minutes. Following lysis, the supernatant was removed from the solution by centrifugation, and the Wizard Genomic DNA Purification Kit (manufactured by Promega) was used to extract the genomic DNA. The DNA concentration was measured using Qubit (manufactured by Thermo Fisher Scientific), and the genomic DNA sample was obtained by diluting to 0.1 ng/µL with ultrapure water.
(2) From the genomic DNA sample obtained in the above (1), a library was prepared using the Nextera XT DNA Library Prep kit (manufactured by Illumina) and purified using AMPure XP (manufactured by Beckman Coulter Life Sciences). Subsequently, the average library size was measured using the High Sensitivity DNA kit (manufactured by Agilent Technologies), and the concentration was measured using the Quan-iT dsDNA High Sensitivity Assay kit (manufactured by Thermo Fisher Scientific). The DNA concentration was diluted to 1.1 nM with ultrapure water, and equal amounts of all the samples were mixed. Then, 5 µL of 1 N sodium hydroxide solution was added to 45 µL of the mixed solution for denaturation. After denaturation, 10 µL of the DNA was mixed with 990 µL of HT1 buffer (manufactured by Illumina), followed by equal amount mixing with 0.2 pM PhiX (manufactured by Illumina). Sequencing analysis was conducted using MiSeq Reagent Kit v3 (manufactured by Illumina, 600 cycles) on the next-generation sequencer MiSeq sequencer (manufactured by Illumina).
(3) The sequence data obtained in the above (2) were transferred to CLC Genomics Workbench (manufactured by QIAGEN) to prepare contigs and annotate them. Using the contig of the P2101201 strain, which was a high acid-producing lactic acid bacterium in Test Example 1, as the reference sequence, the contigs of low acid-producing lactic acid bacteria strains OLS4801, OLS4802, and OLS4803 were mapped to identify gene mutation locations, and only mutation locations accompanied by amino acid mutations were extracted. Table 3 below shows the gene names of the extracted gene mutation locations in the three strains of low acid-producing lactic acid bacteria, the number of bases and the number of amino acids encoded by that base sequence, the mutation locations and types of bases, and the amino acid mutation locations, with the strains from which these mutations were extracted indicated by black circles.

**[Table 3]**

| Gene Name | Number of bases (bp) | Number of Amino acids | Base Mutation | Mutation Type | Amino Acid Mutation | Lactic Acid Bacteria | | |
|---|---|---|---|---|---|---|---|---|
| | | | | | | OLS 4801 | OLS 4802 | OLS 4803 |
| GTP diphosphokinase | 756 | 252 | 426delA | Deletion | 143Asp FS | ● | | |
| ATP synthase epsilon chain | 447 | 149 | 258delA | Deletion | 86Glu FS | | | ● |
| | | | G89C | One Base Substitution | Gly30Ala | ● | ● | |
| 16S rRNA methyltransferase | 591 | 197 | T464C | One Base Substitution | Leu155Ser | ● | ● | ● |
| Transcriptional regulator LacI family | 966 | 322 | 177_178 insA | Insertion | Ala60 FS | ● | ● | ● |
| Insulinase family protein | 1251 | 417 | T338A | One Base Substitution | Leu113His | ● | ● | ● |
| Adenylate kinase | 657 | 219 | G67A | One Base Substitution | Glu23Lys | | ● | |

As shown in Table 3, as a result of the above (3), four genes (ATP synthase epsilon chain gene (ATP synthase complex ε subunit gene (atpC)), 16S rRNA methyltransferase gene, transcriptional regulator LacI family gene, and Insulinase family protein gene) were confirmed to have mutations common to the three strains of low acid-producing lactic acid bacteria.

### (Test Example 3)

Firstly, from Test Example 2, it was examined whether the ATP synthase epsilon chain would contribute to low acid production. Specifically, it was verified whether low acid production would be lost in the recombinant strain prepared by introducing the sequence of the ATP synthase epsilon chain gene of the P2101201 strain, a high acid-producing lactic acid bacterium, into the OLS4803 strain, a low acid-producing lactic acid bacterium.
(1) The recombinant strain of the OLS4803 strain was prepared by double crossover through homologous recombination. Specifically, first, from the genomic DNA extracted from the P2101201 strain, fragments including the full length of the ATP synthase epsilon chain gene were amplified by PCR method using the combination of primers shown in Table 4 below. Next, the obtained fragment was inserted into the temperature-sensitive vector pTERM09 to prepare the recombinant plasmid DNA. Note that pTERM09 possesses a characteristic that allows replication at low temperature (30°C) but not at high temperature (37°C), and harbors the erythromycin resistance gene. The prepared plasmid DNA was introduced into the OLS4803 strain by electroporation, and the transformants were obtained at 30°C. The obtained transformants were cultured under selective pressure with the addition of erythromycin (25 µg/mL) at 37°C, and strains that incorporated the plasmid DNA into the genomic DNA of the OLS4803 strain through homologous recombination were obtained. Next, they were cultured without applying selective pressure with the addition of erythromycin at 30°C, and by conducting the second homologous recombination, the sequence of the P2101201 strain derived from the plasmid DNA and the sequence of the OLS4803 strain on the genomic DNA were swapped to obtain the recombinant strain (OLS4803 strain where the sequence of the ATP synthase epsilon chain gene was recombined with the sequence of the P2101201 strain, Comparative Example 2). The obtained recombinant strain was frozen and stored at -80°C until use.

**[Table 4]**

| Recombination Target Gene | Primer Name | Nucleotide Sequence (5'-3') | SEQ ID NO |
|---|---|---|---|
| ATP synthase epsilon chain | SNV F | TGAGGTGAAGATATGGCACA | 19 |
| | SNV R | TCTATTACGTTACCCTACATTTC | 20 |

(2) The reactivation medium was prepared by partially sterilizing 10% reduced skim milk comprising 0.1 w/v% Meast P2G (manufactured by Asahi Food & Healthcare, Ltd.) at 121°C for 7 minutes. The frozen bacterial solution of the recombinant strain obtained in the above (1) was inoculated into the reactivation medium to 1% by mass and was reactivated and cultured twice under anaerobic conditions at 37°C for 16 hours. Then, it was inoculated into a 10% skimmed milk powder medium that had been partially sterilized to reach 95°C, such that the mass of the post-reactivation culture solution was 2% by mass, and static culture (fermentation) was carried out at 43°C. The time from the inoculation until the pH of the culture solution dropped to 4.6 (fermentation time) was measured. Furthermore, the culture was terminated when the pH of the culture solution reached 4.6, immediately cooled to 10°C, hermetically sealed to prevent air entry, and stored statically for 1 day, 7 days, 14 days, or 21 days, and the pH after each storage period (10°C pH) was measured. Also, by spreading a diluted suspension of each post-storage culture solution on BCP added plate count agar medium (manufactured by Eiken Chemical Co., Ltd.) and culturing it, the number of colonies appearing was counted, and the viable bacterial count (cfu/g) in each culture solution was measured.

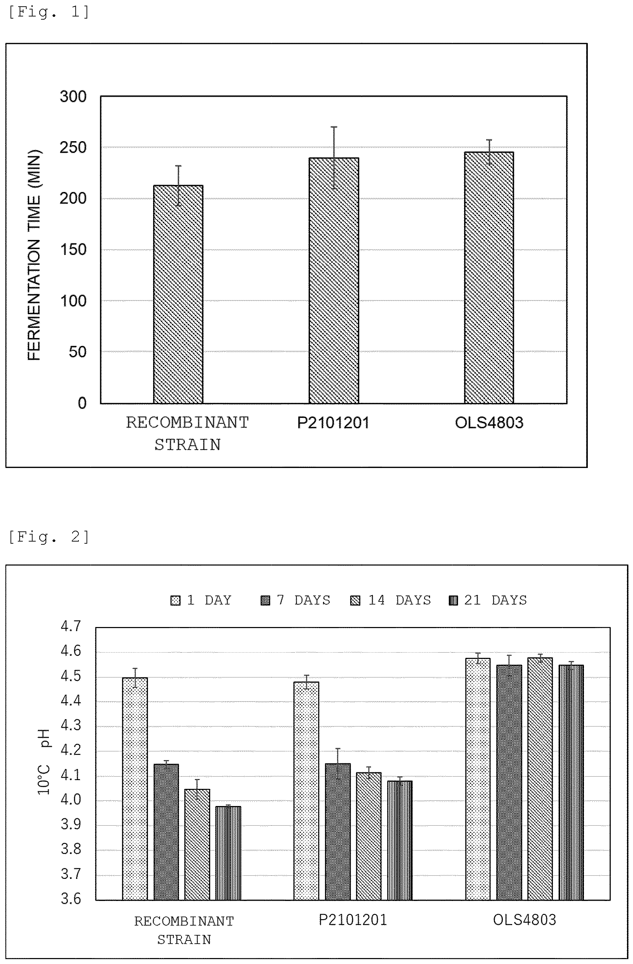

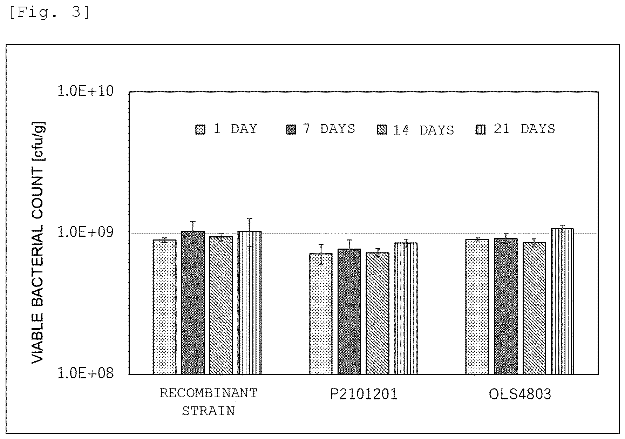

The fermentation time (minutes) of the recombinant strain is shown in Fig. 1, the pH (10°C pH) at each storage period after 10°C storage is shown in Fig. 2, and the viable bacterial count (cfu/g) at each storage period after 10°C storage is shown in Fig. 3. Figs. 1 to 3 also show results of similar fermentation time measurement, 10°C post-storage pH measurement, and 10°C post-storage viable bacterial count measurement for the non-recombined P2101201 strain and OLS4803 strain. Note that although measurements were conducted for 10 obtained recombinant strains, since there were no differences in the results among the 10 strains, Figs. 1 to 3 only show the results of one strain (No. 1) out of the 10 strains.

As shown in Fig. 1, fermentation was completed (pH reached 4.6) within 5 hours in the recombinant strain, the P2101201 strain, and the OLS4803 strain. On the other hand, as shown in Fig. 2, regarding the time course in pH with storage at 10°C, in the recombinant strain, similar to the P2101201 strain, the pH dropped below 4.2 after 7 days of storage, and the low acid production observed in the non-recombined OLS4803 strain was lost. Therefore, it was confirmed that mutations in the ATP synthase epsilon chain gene and the amino acid sequence encoded by it contributed to low acid production in the OLS4803 strain.

Furthermore, as shown in Fig. 3, regarding the time course in viable bacterial count with storage at 10°C, there was almost no difference between the recombinant strain, the P2101201 strain, and the OLS4803 strain, and it was confirmed that the viable bacterial count was maintained even by low-temperature storage regardless of the presence or absence of the mutation. Therefore, it can be said that the low acid production is not exhibited through apoptosis.

### (Test Example 4)

Following Test Example 2, it was examined whether the other three genes (16S rRNA methyltransferase gene, transcriptional regulator LacI family gene, and Insulinase family protein gene) would contribute to low acid production. Specifically, it was verified whether low-acid production would be exhibited in the recombinant strain prepared by introducing the sequence of each gene from the OLS4803 strain, which was a high acid-producing lactic acid bacterium, into the P2101201 strain, which was a low acid-producing lactic acid bacterium.

First, the recombinant strain of the P2101201 strain was prepared by double crossover through homologous recombination. Specifically, from the genomic DNA extracted from the OLS4803 strain, fragments including the full length of each gene were amplified by PCR method using the combination of primers shown in Table 5 below. Apart from using the obtained fragments to introduce the prepared plasmid DNA into the P2101201 strain, each recombinant strain (P2101201 strain introduced with the sequence of each gene of the OLS4803 strain) was obtained, similarly as in (1) of Test Example 3, 7 strains for each (No. 1-7).

**[Table 5]**

| Recombination Target Gene | Primer Name | Nucleotide Sequence (5'-3') | SEQ ID NO |
|---|---|---|---|
| 16S rRNA methyltransferase | 16S-F | CACCGGATACATACCCTCGA | 21 |
| | 16S-R | TCCCTCACTTCCTGAACGTC | 22 |
| Transcriptional regulator LacI family | LacI-F | ATGGTCGCAAAGCTTACTGA | 23 |
| | LacI-R | CAGTTCGTGACAGACGAAGC | 24 |
| Insulinase family protein | Insulinase-F | AGCGATTATCTAGTGCCCCA | 25 |
| | Insulinase-R | GCCCTTAATAGCTCCACCAC | 26 |

Next, for each obtained recombinant strain, culture (fermentation) was carried out similarly as in (2) of Test Example 3, and the time from the inoculation until the pH of the culture solution dropped to 4.6 (fermentation time) was measured. Furthermore, the culture was terminated when the pH of the culture solution reached 4.6, immediately cooled to 10°C, hermetically sealed to prevent air entry, and stored statically for 6 days, 7 days, 8 days, 14 days, 15 days, 33 days, or 34 days, and the pH after each storage period (10°C pH) was measured. The results of the recombinant strain of the 16S rRNA methyltransferase gene are shown in Table 6, the results of the recombinant strain of the transcriptional regulator LacI family gene are shown in Table 7, and the results of the recombinant strain of the Insulinase family protein gene are shown in Table 8. Further, in Tables 6 to 8, the results of the same fermentation time measurement and pH measurement after storage at 10°C are also shown for the unrecombined P2101201 strain and OLS4803 strain.

**[Table 6]**

| 16S rRNA methyltransferase | | | | | |
|---|---|---|---|---|---|
| Lactic Acid Bacteria | | Fermentation Time (Min) | 10°C pH | | |
| | | | 7 Days | 15 Days | 34 Days |
| Recombinant Strain | No. 1 | 277 | 4.12 | 4.05 | 4.00 |
| | No. 2 | 268 | 4.10 | 4.00 | 3.98 |
| | No. 3 | 274 | 4.07 | 4.01 | 3.96 |
| | No. 4 | 281 | 4.05 | 4.02 | 3.97 |
| | No. 5 | 265 | 4.06 | 4.04 | 3.98 |
| | No. 6 | 287 | 4.10 | 4.06 | 4.01 |
| | No. 7 | 268 | 4.06 | 4.01 | 3.97 |
| Recombinant Strain Avg | | 274 | 4.08 | 4.03 | 3.98 |
| P2101201 | | 274 | 4.19 | 4.08 | 4.05 |
| OLS4803 | | 281 | 4.53 | 4.53 | 4.51 |

**[Table 7]**

| Transcriptional regulator LacI family | | | |
|---|---|---|---|
| Lactic Acid Bacteria | | Fermentation Time (Min) | 10°C pH |
| | | | 8 Days |
| Recombinant Strain | No. 1 | 266 | 4.16 |
| | No. 2 | 269 | 4.12 |
| | No. 3 | 261 | 4.13 |
| | No. 4 | 252 | 4.11 |
| | No. 5 | 261 | 4.12 |
| | No. 6 | 276 | 4.13 |
| | No. 7 | 259 | 4.11 |
| Recombinant Strain Avg | | 263 | 4.13 |
| P2101201 | | 252 | 4.18 |
| OLS4803 | | 289 | 4.56 |

**[Table 8]**

| Insulinase family protein | | | | | |
|---|---|---|---|---|---|
| Lactic Acid Bacteria | | Fermentation Time (Min) | 10°C pH | | |
| | | | 6 Days | 14 Days | 33 Days |
| Recombinant Strain | No. 1 | 240 | 4.13 | 4.01 | 3.97 |
| | No. 2 | 290 | 4.13 | 4.03 | 4.02 |
| | No. 3 | 262 | 4.15 | 4.07 | 4.03 |
| | No. 4 | 266 | 4.14 | 4.05 | 4.03 |
| | No. 5 | 283 | 4.15 | 4.05 | 4.01 |
| | No. 6 | 283 | 4.16 | 4.06 | 4.02 |
| | No. 7 | 283 | 4.16 | 4.06 | 3.99 |
| Recombinant Strain Avg | | 272 | 4.15 | 4.05 | 4.01 |
| P2101201 | | 250 | 4.08 | 4.00 | 3.96 |
| OLS4803 | | 272 | 4.48 | 4.49 | 4.47 |

As shown in Tables 6 to 8, regarding the time course in pH with storage at 10°C, there was almost no difference between each recombinant strain and the P2101201 strain, and low-acid production as in the OLS4803 strain was not exhibited in any of the recombinant strains. Therefore, it has been confirmed that the aforementioned three genes (16S rRNA methyltransferase gene, transcriptional regulator LacI family gene, and Insulinase family protein gene) do not contribute to low acid production, and what contributes to the low acid production of lactic acid bacteria is a mutation in the ATP synthase epsilon chain gene and the amino acid sequence encoded by it.

### [Industrial Applicability]

As described above, the present invention makes it possible to provide lactic acid bacterium which allows for obtaining fermented milk in which the decrease in pH during low-temperature storage is suppressed, and the viable bacterial count of the lactic acid bacteria is sufficiently maintained even by the low-temperature storage, a lactic acid bacterium starter and fermented milk comprising the same, and a method for producing fermented milk, and further a method for screening lactic acid bacterium.

### [Accession Number]

1.
   (1) Identification label: Streptococcus thermophilus OLS4801
   (2) Accession number: NITE BP-03504
   (3) Accession date: August 4, 2021
   (4) Depositary institution: National Institute of Technology and Evaluation NITE Patent Microorganisms Depositary
2.
   (1) Identification label: Streptococcus thermophilus OLS4802
   (2) Accession number: NITE BP-03505
   (3) Accession date: August 4, 2021
   (4) Depositary institution: National Institute of Technology and Evaluation NITE Patent Microorganisms Depositary
3.
   (1) Identification label: Streptococcus thermophilus OLS4803
   (2) Accession number: NITE BP-03506
   (3) Accession date: August 4, 2021
   (4) Depositary institution: National Institute of Technology and Evaluation NITE Patent Microorganisms Depositary
4.
   (1) Identification label: Streptococcus thermophilus OLS4824
   (2) Accession number: NITE BP-03508
   (3) Accession date: August 4, 2021
   (4) Depositary institution: National Institute of Technology and Evaluation NITE Patent Microorganisms Depositary
5.
   (1) Identification label: Streptococcus thermophilus OLS4496 strain
   (2) Accession number: NITE BP-02875
   (3) Accession date: February 5, 2019
   (4) Depositary institution: National Institute of Technology and Evaluation NITE Patent Microorganisms Depositary

## Claims

1. A lactic acid bacterium in which an ATP synthase complex is functionally inhibited.

2. The lactic acid bacterium according to claim 1, wherein an ε subunit of the ATP synthase complex is functionally inhibited.

3. The lactic acid bacterium according to claim 1, which is a lactic acid bacterium of the genus Streptococcus.

4. The lactic acid bacterium according to claim 3, which is a lactic acid bacterium belonging to Streptococcus thermophilus.

5. The lactic acid bacterium according to claim 4, possessing a prtS gene.

6. The lactic acid bacterium according to claim 1, wherein the function inhibition of the ATP synthase complex is a loss-of-function mutation in an ε subunit gene.

7. The lactic acid bacterium according to claim 1, wherein the function inhibition of the ATP synthase complex is a decreased expression of an ε subunit protein.

8. A lactic acid bacterium composition comprising: the lactic acid bacterium according to claim 1.

9. The lactic acid bacterium composition according to claim 8, wherein the lactic acid bacterium is a lactic acid bacterium of the genus Streptococcus, the composition further comprising: lactic acid bacterium of the genus Lactobacillus.

10. The lactic acid bacterium composition according to claim 8, which is a lactic acid bacterium starter.

11. The lactic acid bacterium composition according to claim 8, which is fermented milk.

12. A method for producing fermented milk comprising: a fermentation step of adding the lactic acid bacterium according to claim 1 or the lactic acid bacterium composition according to claim 8 to a formula milk comprising raw material milk, followed by fermentation to thereby obtain fermented milk.

13. The method for producing fermented milk according to claim 12, wherein the lactic acid bacterium is a lactic acid bacterium of the genus Streptococcus, the method further comprising: adding lactic acid bacterium of the genus Lactobacillus to the formula milk.

14. A method for screening lactic acid bacterium, comprising: a selection step of selecting lactic acid bacterium with low acid production using function inhibition of an ATP synthase complex as an indicator.

15. A method for producing fermented milk comprising: a fermentation step of adding lactic acid bacterium selected by the method for screening lactic acid bacterium according to claim 14 to a formula milk comprising raw material milk, followed by fermentation to thereby obtain fermented milk.
